# EUROPÄISCHE PATENTANMELDUNG

(11) **EP 2 078 714 A2**
(43) Veröffentlichungstag der Anmeldung: **15.07.2009**
(21) Anmeldenummer: 08167890.6
(22) Anmeldetag: 30.10.2008
(51) Int. Cl.: C07D 233/06, C07D 233/14, C10M 133/00

(54) **Imidazolinium-Salze mit niedrigem Schmelzpunkt, Verfahren zu deren Herstellung und deren Verwendung als Schmiermittel**

(30) Priorität: 29.12.2007 DE 102007063149
(71) Anmelder: Evonik Goldschmidt GmbH, 45127 Essen (DE)
(72) Erfinder: Schwab, Peter, 45133, Essen (DE); Köhle, Hans-Jürgen, 63533, Mainhausen (DE); Salomon, Thomas, 63628, Bad Soden-Salmünster (DE); Hell, Kerstin, 45326, Essen (DE)

(57) **Zusammenfassung**

Die Erfindung betrifft neuartige Imidazolinium-Salze, Verfahren zu deren Herstellung und die Verwendung der Imidazolinium-Salze oder einer Mischung von Imidazolinium-Salzen mit weiteren (Schmier-) Stoffen als Schmiermittel.

## Beschreibung

Die Erfindung betrifft neuartige Imidazolinium-Salze mit niedrigen Schmelzpunkten sowie ein Verfahren zu deren Herstellung. Sie betrifft ferner die Verwendung dieser Stoffe als Basisöl oder als Additiv in Schmiermitteln.

### Stand der Technik:

Schmierstoffe (auch: Schmiermittel) werden zur Schmierung eingesetzt und dienen zur Verringerung von Reibung und Verschleiß, sowie zur Kraftübertragung, Kühlung, Schwingungsdämpfung, Dichtwirkung und dem Korrosionsschutz.

Während jeder Bewegung kommt es zu einer Relativbewegung zwischen Bauteilen und damit zu einer Reibung zwischen den Oberflächen. Um Energieverlust und Verschleiß zu minimieren, ist es daher erforderlich, zwischen den bewegten Bauteilen ein Schmiermittel aufzubringen. Zur Schmierung werden Mineralöle, Poly-alpha-olefine, native Öle (z.B. Rapsöle), synthetische Esteröle und niedrigviskose Polyglykole eingesetzt.

Schmieröle sind die wichtigsten technischen Schmierstoffe. Sie dienen zur Verringerung von Reibung, die Geräuschentwicklung und besonders Materialverschleiß verursacht. Darüber hinaus ermöglicht die Verwendung von Schmieröl auch die Wärmeabfuhr. Schmieröl bildet zwischen bewegten Flächen, etwa in einem Scharnier, einen Gleitfilm.

Die ersten synthetischen Schmierstoffe wurden in den 1930er und 40er Jahren entwickelt. Ausgangspunkte waren Versuche mit Erdöl und diversen Additiven und später Synthetisierung durch Ethylen. Die Bedeutung dieser synthetischen Schmieröle lag u.a. in der Veränderung und Abhängigkeit der Viskosität auch unter extremen Temperaturbedingungen. Diese Arbeiten führten zu der Herstellung von über 3.500 Estern in den genannten Jahren, darunter auch Diester und Polyol Ester.

Moderne Viertaktmotoren-Öle sind in der Regel Mineral- oder Synthetikgrundöle mit einem Additivpaket. Das Grundöl ist beim Mineralöl ein Erdöldestillat. Die Destillate werden danach unter anderem noch gefiltert, geklärt und raffiniert, sodass man ein unlegiertes Öl mit einer bestimmten Viskosität erhält. Dieses Öl ist kein reiner Stoff, sondern eine Fraktion, also ein Gemisch unterschiedlicher Kohlenwasserstoffe mit ähnlichem Siedebereich. Diese Öle waren lange Zeit die einzigen in KFZ verwendeten Öle, heute sind sie von einigen Herstellern noch als Kompressorenöl oder Maschinenöl im Angebot (sehr alte Motoren (Vorkriegsmaschinen) benötigen diese Öle, da deren Dichtungsmaterialien oft nicht mit den modernen Additiven verträglich sind).

Bis in die vierziger Jahre und danach wurden hochbelastete Motoren (vor allem Motorradrennmotoren) auch noch mit Pflanzenöl (Rizinusöl) geschmiert.
Bereits in der Vergangenheit wurden diverse Mittel angeboten, welche die Ölqualität der Mineralöle verbessern sollten; einige davon gibt es noch heute. Neben Festkörperzusätzen wie Molybdändisulfid und Kolloidgraphit (Kugelgraphit) gab es auch verschiedene chemische Zusätze, die teilweise ihren Zweck recht gut erfüllten. Ungefähr in den 1940er Jahren kamen Öle auf den Markt, die serienmäßig mit derartigen Zusätzen ausgestattet waren und als HD-Öle (heavy duty - hohe Beanspruchung) vermarktet wurden.

Verbrennungsmotoren stellen hohe Ansprüche an das Motoröl. Das Motoröl ist nicht nur Schmierstoff, sondern hat weitere wichtige motorische Aufgaben, nämlich:
- Übertragung von Kräften (hydraulisch in Kettenspannern und Stößel),
- Verschleißschutz (der sich gegeneinander bewegenden Motorteile),
- Korrosionsschutz der Motorteile gegenüber aggressiven Verbrennungsprodukten durch Bildung von Schutzschichten auf der Metalloberfläche,
- Abdichten (des Brennraums zum Kurbelgehäuse, der Ansaug- und Abgaskanäle über die Ventilführungen zum Ventiltrieb),
- Kühlen (von v. a. Kolben und Kurbelwelle),
- Neutralisation von sauren Verbrennungsprodukten durch chemische Umwandlung,
- Reinhaltung der Motorenteile durch Ablösen von Verbrennungsrückständen (und Alterungsprodukten des Motoröls) mit öllöslichen Seifen,
- Schmierung (Trennung der sich gegeneinander bewegenden Metallflächen),
- Dispergieren von festen Fremdstoffen, Staub, Abrieb, Verbrennungsprodukte wie Ruß oder Asche,

Um diese Aufgaben erfüllen zu können, werden vielerlei Anforderungen an das Motoröl gestellt, die durch chemische, physikalische und technologische Eigenschaften charakterisiert sind. Diese Eigenschaften sind vereinfacht:
- Viskosität und Fließverhalten,
- Oberflächenaktives Verhalten,
- Neutralisationsvermögen,

Daneben werden folgende Anforderungen an das Motorenöl gestellt:
- Neutrales Verhalten gegenüber Dichtungswerkstoffen,
- Geringe Schaumneigung.
- Lange Gebrauchsdauer, lange Ölwechselintervalle,
- Niedriger Ölverbrauch,
- Niedriger Kraftstoffverbrauch,
- Kraftstoffverträglichkeit,
- Umweltverträglichkeit.

Diese vorbekannten Schmierstoffe weisen jeweils unterschiedliche Nachteile auf. Native Öle weisen erhebliche Mängel im Kälteverhalten, in der UV-Stabilität, im Alterungsverhalten, sowie in der Temperaturbeständigkeit und Wasserbeständigkeit auf. Mineralöle haben niedrige Viskositätsindices, hohe Verdampfungsverluste, geringe Kälteeignung und mäßige thermische Belastbarkeit. Polyglykole zeigen eine niedrige thermische Stabilität, einen hohen Verdampfungsverlust, niedrige Viskositäten und schlechte Dichtungsverträglichkeiten. Esteröle sind nicht hydrolysestabil und zeigen niedrige Scherstabilitäten. Alle vorbenannten Schmierstoffe laden sich beim Filtrierprozess, insbesondere bei niedrigen Temperaturen statisch auf, was zu gefährlichen elektrischen Entladungen führen kann.

Generell ist bei der Wahl der Schmiermittel darauf zu achten, dass der Stoff sehr niedrige Reibungskoeffizienten, ausreichendes Kältefließvermögen (bis -60 °C), gute thermische Beständigkeit (bis 400 °C), gute Lack- und Dichtungsverträglichkeit, niedrige Toxizität und Ecotoxizität, hohe Scherstabilität, hohe Viskositätsindices, niedrige Korrosivität, gute Additivierbarkeit und sehr guten Verschleißschutz besitzt.

### Aufgabenstellung:

Aufgabe der Erfindung ist es daher, ein Schmiermittel für die Schmierung von beweglichen Teilen bereit zu stellen, welches ein verbessertes Eigenschaftsprofil bietet, um insbesondere über einen breiten Temperaturbereich einen sehr niedrigen Reibungsverlust (fuel efficiency) zu ermöglichen.

Dies wird erfindungsgemäß mit einer Verwendung von mindestens einem neuartigen Imidazolinium-Salz oder einer Mischung von neuartigen Imidazolinium-Salzen oder einer Beimischung von neuartigen Imidazolinium-Salzen zu anderen Schmiermitteln erreicht.

Gegenstand der Erfindung sind daher neuartige 1-Hydroxyalkyl-imidazolinium- und 1-Alkoxyalkyl-imidazolinium-Salze der allgemeinen Formel (I) 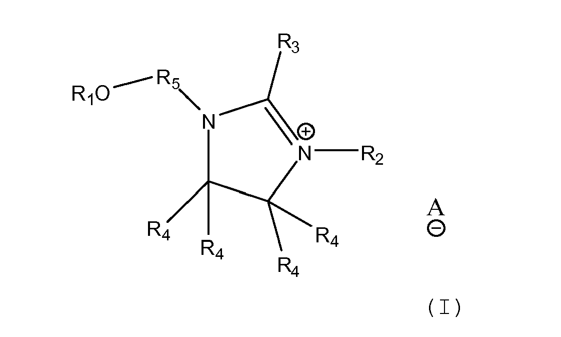 wobei
- R₁: Wasserstoff oder ein gegebenenfalls verzweigtes, gegebenenfalls ungesättigtes, gegebenenfalls Sauerstoffatome oder Stickstoffatome enthaltendes Alkylradikal mit 1 bis 20 C-Atomen, bevorzugt mit 1 bis 12 C-Atomen,
- R₂: Wasserstoff oder ein gegebenenfalls verzweigtes, gegebenenfalls ungesättigtes, gegebenenfalls Sauerstoffatome oder Stickstoffatome enthaltendes Alkylradikal mit 1 bis 20 C-Atomen, bevorzugt mit 1 bis 12 C-Atomen, besonders bevorzugt mit 1 bis 4 C-Atomen,
- R₃: ein gegebenenfalls verzweigtes, gegebenenfalls ungesättigtes, gegebenenfalls von Sauerstoffatomen unterbrochenes Alkylradikal mit 1 bis 20 C-Atomen, bevorzugt mit 1 bis 12 C-Atomen,
- R₄: jeweils unabhängig voneinander Wasserstoff oder Alkyl, bevorzugt Butyl, Propyl, Ethyl, Methyl oder Wasserstoff,
- R₅: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Ethylen, ist und
- A: ein Gegenion zu den positiven Ladungen an den quartären Stickstoffgruppen sind,
sowie 1,3-Dihydroxyalkyl-imidazolinium- und 1-Alkoxyalkyl-3-hydroxy-imidazolinium-Salze der allgemeinen Formel (II), 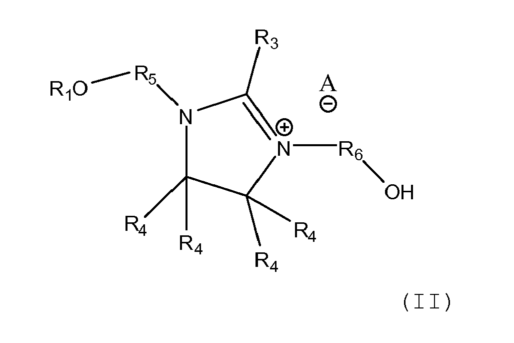 wobei
- R₁, R₃, R₄ und R₅: die unter Formel (I) genannten Bedeutungen besitzen und
- R₆: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Ethylen, ist und
A ein Gegenion zu den positiven Ladungen an den quartären Stickstoffgruppen ist, sowie der allgemeinen Formel (III) 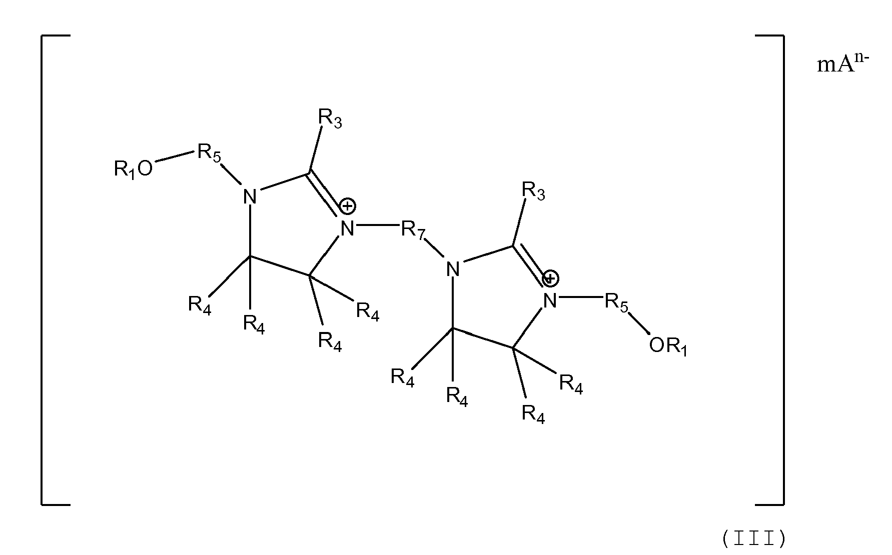
wobei
- R₁, R₃, R₄ und R₅: die unter Formel (I) genannten Bedeutungen besitzen und
- R₇: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Butylen, Propylen, Ethylen, Methylen, besonders bevorzugt, Butylen und Ethylen, ist,
- A: ein Gegenanion zu den positiven Ladungen an den quartären Stickstoffgruppen ist und die Multiplikation aus m und n gleich der Ladung des Imidazoliniumkations entspricht; bevorzugt ist m und/oder n gleich 1 oder 2.

Die neuartigen Imidazolinium-Salze besitzen als Anionen [A]ⁿ⁻ bevorzugt solche, die dem anspruchsvollen Anwendungsgebiet als Schmiermittel genügen und beispielsweise eine hohe Temperaturresistenz aufweisen und somit weder die Viskositätseigenschaften, noch die Schmelzeigenschaften der Salze negativ beeinflussen, wie zum Beispiel ausgewählt aus den Gruppen der Halogenide, Carboxylate, Phosphate, Thiocyanate, Isothiocyanate, Dicyanamide, Sulfate, Alkylsulfate, Sulfonate, Alkylsulfonate, Tetrafluoroborate, Hexafluoro-phosphate oder auch Bis(trifluormethylsulfonyl)imide, wobei die vorgenannten Anionen eine kleine Auswahl aus der großen Zahl möglicher Anionen darstellen und damit kein Anspruch auf Vollständigkeit erhoben oder gar eine Einschränkung vorgegeben werden soll.

Die erfindungsgemäß bevorzugt eingesetzten Imidazolinium-Salze bestehen aus mindestens einem der vorgenannten Kationen der Formel (I), (II) oder (III) kombiniert mit jeweils mindestens einem Anion [A]ⁿ⁻.

Den Imidazoliniumsalzen der Formeln (I), (II) und (III) ist gemeinsam das Imidazoliniumgerüst, das im Wesentlichen die Produkteigenschaften in Hinblick auf den Verwendungszweck als Schmiermittel oder Schmiermittelbestandteil beeinflusst. Ob das Imidazoliniumgerüst dabei unterschiedlich substituiert ist oder gar in Form einer dimeren Struktur oder gar oligomeren Form vorliegt, scheint eine untergeordnete Rolle zu spielen. Wichtig für die Produkteigenschaften sind beispielsweise die Schmelzpunkte oder Schmelzbereiche der Verbindungen, deren Viskositäts-Temperatur-Verhalten über einen großen Temperaturbereich, ihre niedrigen Reibungskoeffizienten, ihre thermische Stabilität gegenüber Zersetzung sowie ihre ausgewogene Hydrophilie, die eine verbesserte Handhabbarkeit gegenüber den marktbeherrschenden, zumeist hydrophoben Schmiermittel auszeichnet.

Das Anion [A]ⁿ⁻ des Imidazolinium-Salzes ist beispielsweise ausgewählt aus:
- der Gruppe der Halogenide, Pseudohalogenide, Mineralsäureanionen und halogenhaltigen Verbindungen der Formeln: F⁻ Cl⁻, Br⁻, I⁻, BF₄⁻, PF₆⁻, BCl₄⁻ , CF₃SO₃⁻, (CF₃SO₃)₂N⁻, CF₃CO₂⁻, CCl₃CO₂⁻, CN⁻, SCN⁻, OCN⁻, NO₂⁻, NO₃⁻, N(CN)⁻;
- der Gruppe der Sulfate, Sulfite und Sulfonate der allgemeinen Formeln: SO₄²⁻, HSO₄⁻, SO₃^{2"}, HSO₃⁻, R^{a}OSO₃⁻, R^{a}SO₃⁻;
- der Gruppe der Phosphate der allgemeinen Formeln: PO₄³⁻, HPO₄²⁻, H₂PO⁴⁻, R^{a}PO₄²⁻, HR^{a}PO₄-_{,} R^{a}R^{b}PO₄⁻;
- der Gruppe der Phosphonate und Phosphinate der allgemeinen Formel: R^{a}HPO₃⁻, R^{a}R^{b}PO₂⁻, R^{a}R^{b}PO₃⁻;
- der Gruppe der Phosphite der allgemeinen Formeln: PO₃³⁻, HPO₃²⁻, H₂PO₃⁻, R^{a}PO₃²⁻, R^{a}HPO₃⁻, R^{a}R^{b}PO₃⁻;
- der Gruppe der Phosphonite und Phosphinite der allgemeinen Formel: R^{a}R^{b}PO₂⁻, R^{a}HPO₂⁻, R^{a}R^{b}PO⁻, R^{a}HPO⁻;
- der Gruppe der Carboxylate der allgemeinen Formeln: R^{a}COO⁻;
- der Gruppe der Borate der allgemeinen Formeln: BO₃³⁻, HBO₃²⁻, H₂BO₃⁻, R^{a}R^{b}BO₃⁻, R^{a}HBO₃⁻, R^{a}BO₃²⁻, B(OR^{a}) (OR^{b}) (OR^{c}) (OR^{d})⁻_{,} B(HSO₄)⁻, B(R^{a}SO₄)⁻;
- der Gruppe der Boronate der allgemeinen Formeln: R^{a-}BO₂²⁻, R^{a}R^{b}BO⁻;
- der Gruppe der Carbonate und Kohlensäureester der allgemeinen Formeln: HCO₃⁻, CO₃²⁻, R^{a}CO₃⁻;
- der Gruppe der Silikate und Kieselsäuresäureester der allgemeinen Formeln: SiO₄⁴⁻, HSiO₄³⁻, H₂SiO₄²⁻, H₃SiO₄⁻, R^{a}SiO₄³⁻, R^{a}R^{b}SiO₄²⁻, R^{a}R^{b}R^{c}SiO₄⁻, HR^{a}SiO₄²⁻, H₂R^{a}SiO₄⁻, HR^{a}R^{b}SiO₄⁻;
- der Gruppe der Alkyl- bzw. Arylsilan-Salze der allgemeinen Formeln: R^{a}SiO₃³⁻, R^{a}SiO₂²⁻, R^{a}R^{b}R^{c}SiO⁻, R^{a}R^{b}R^{c}Si0₃⁻, R^{a}R^{b}R^{c}Si0₂⁻, R^{a}R^{b}Si0₃²⁻;
- der Gruppe der Carbonsäureimide, Bis(sulfonyl)imide und Sulfonylimide der allgemeinen Formeln: 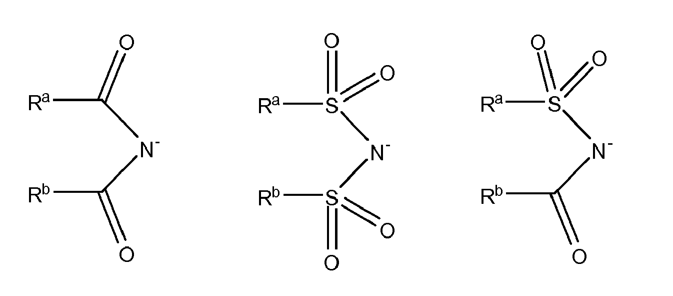
- der Gruppe der Methide der allgemeinen Formel: 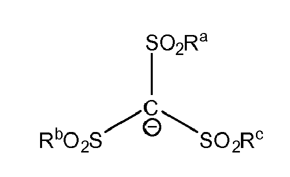
- der Gruppe der Alkoxide und Aryloxide der allgemeinen Formeln: R^{a}O⁻;
- der Gruppe der Halometallate der allgemeinen Formel [MᵣHalₜ]^{s-}, wobei M für ein Metall und Hal für Fluor, Chlor, Brom oder Iod steht, r und t ganze positive Zahlen sind und die Stöchiometrie des Komplexes angeben und s eine ganze positive Zahl ist und die Ladung des Komplexes angibt; wie beispielsweise AlCl₄⁻, Al₂Cl₇⁻, Al₃Cl₁₀⁻, AlBr₄⁻, FeCI₄⁻, SbF₆⁻, AsF₆⁻, ZnCl₃⁻, SnCl₃⁻, CuCl₂⁻,
- der Gruppe der Sulfide, Hydrogensulfide, Polysulfide, Hydrogenpolysulfide und Thiolate der allgemeinen Formeln:
   S²⁻, HS⁻, [Sᵥ]²⁻, [HSᵥ]⁻, [R^{a}S]⁻, wobei v eine ganze positive Zahl von 2 bis 10 ist;
- der Gruppe der komplexen Metallionen wie Fe(CN)₆³⁻, Fe(CN)₆⁴⁻, MnO₄⁻, Fe(CO)₄⁻.

Darin bedeuten R^{a}, R^{b}, R^{c} und R^{d} unabhängig voneinander jeweils
- Wasserstoff;
- C₁-C₃₀-Alkyl und deren aryl-, heteroaryl-, cycloalkyl-, halogen-, hydroxy-, amino-, carboxy-, formyl-, -O-, -CO-, -CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2- pentyl, 2-Methyl-3-pentyl, 3-Methyl-3-pentyl, 2,2-Dimethyl-1-butyl, 2,3-Dimethyl- 1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Henicosyl, Docosyl, Tricosyl, Tetracosyl, Pentacosyl, Hexacosyl, Heptacosyl, Octacosyl, Nona-cosyl, Triacontyl, Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclo-pentylpropyl, Cyclohexylmethyl, 2-Cyclohexylethyl, 3-Cyclohexylpropyl, Methoxy, Ethoxy, Formyl, Acetyl oder C_{q}F_{2(q-a)+(1-b)}H_{2a+b} mit q < 30, 0 ≤ a ≤ q und b = 0 oder 1 (beispielsweise CF₃, C₂F₅, CH₂CH₂-C_{(q-2)}F_{2(q-2)+1}, C₆F₁₃, C₈F₁₇, C₁₀F₂₁, C₁₂F₂₅);
- C₃-C₁₂-Cycloalkyl und deren aryl-, heteroaryl-, cycloalkyl-, halogen-, hydroxy-, amino-, carboxy-, formyl-, -O-, -CO- oder -CO-0-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl o- der C_{q}F_{2(q-a)-(1-b)}H_{2a-b} mit q ≤ 30, 0 ≤ a ≤ q und b = 0 oder 1;
- C₂-C₃₀-Alkenyl und deren aryl-, heteroaryl-, cycloalkyl-, halogen-, hydroxy-, amino-, carboxy-, formyl-, -O-, -CO- oder -CO-0-substituierte Komponenten, wie beispielsweise 2-Propenyl, 3-Butenyl, cis-2-Butenyl, trans-2-Butenyl oder C_{q}F_{2(q-a)-(1-b)}H_{2a-b} mit q ≤ 30, 0 ≤ a ≤ q und b = 0 oder 1;
- C₃-C₁₂-Cycloalkenyl und deren aryl-, heteroaryl-, cycloalkyl-, halogen-, hydroxy-, amino-, carboxy-, formyl-, -O-, -CO- oder -CO-0-substituierte Komponenten, wie beispielsweise 3-Cyclopentenyl, 2-Cyclohexenyl, 3-Cyclohexenyl, 2,5- Cyclohexadienyl oder C_{q}F_{2(q-a)-3(1-b)}H_{2a-3b} mit q ≤ 30, 0 ≤ a ≤ q und b = 0 oder 1;
- Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO- oder -CO-O-substituierte Komponenten, wie beispielsweise Phenyl, 2-Methyl-phenyl (2-ToIyI), 3-Methyl-phenyl (3-ToIyI), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤ a ≤ 5; oder
- zwei Reste einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring.

Ganz besonders bevorzugte Anionen sind zum Beispiel Chlorid; Bromid; Iodid; Thiocyanat; Hexafluorophosphat; Trifluormethansulfonat; Methansulfonat; Formiat; Acetat; Glycolat; Lactat; Mandelat; Nitrat; Nitrit; Trifluoracetat; Sulfat; Hydrogensulfat; Methylsulfat; Ethylsulfat; 1-Propylsulfat; 1-Butylsulfat; 1-Hexylsulfat; 1-Octylsulfat; Phosphat; Dihydrogenphosphat; Hydrogenphosphat; C₁-C₄-Dialkylphosphate; Propionat; Tetrachloroaluminat; Al₂Cl₇⁻; Chlorozinkat; Chloroferrat; Bis(trifluoromethylsulfonyl)imid; Bis(pentafluoroethylsulfonyl) imid; Bis (methylsulfonyl) imid; Bis (p-Tolylsulfonyl)imid; Tris(trifluoromethylsulfonyl)methid; Bis-(pentafluoroethylsulfonyl)methid; p-Tolylsulfonat; Tetracarbonylcobaltat; Dimethylenglykolmonomethylethersulfat; Oleat; Stearat; Acrylat; Methacrylat; Maleinat; Hydrogencitrat; Vinylphosphonat; Bis(pentafluoroethyl)phosphinat; Borate wie Bis[salicylato(2-)]borat, Bis[oxalato(2-)]borat, Bis[1,2-benzoldiolato(2-)-O,O']borat, Tetracyanoborat, Tetrafluoroborat; Dicyanamid; Tris(pentafluoroethyl)trifluorophosphat; Tris(heptafluoropropyl)trifluorophosphat, cyclische Arylphosphate wie Brenzcatecholphosphat (C₆H₄O₂)P(O)O⁻ und Chlorocobaltat.

Bevorzugte Anionen werden ausgewählt aus der Gruppe - ohne Anspruch auf Vollständigkeit - der Halogenide, Bis(perfluoralkylsulfonyl)amide bzw. -imide wie z.B. Bis(trifluormethylylsulfonyl)imid, Alkyl- und Aryltosylate, Perfluoralkyltosylate, Nitrat, Sulfat, Hydrogensulfat, Alkyl- und Arylsulfate, Polyethersulfate und -sulfonate, Perfluoralkylsulfate, Sulfonat, Alkyl- und Arylsulfonate, perfluorierte Alkyl- und Arylsulfonate, Alkyl- und Arylcarboxylate, Perfluoralkylcarboxylate, Perchlorat, Tetrarchloroaluminat, Saccharinat. Weiterhin sind Dicyanamid, Thiocyanat, Isothiocyanat, Tetraphenylborat, Tetrakis(pentafluorphenyl)borat, Tetrafluoroborat, Hexafluorophosphat, Polyetherphosphate und Phosphat bevorzugte Anionen.

Ganz besonders bevorzugte Anionen sind:
Hydrogensulfat, Tetrachloroaluminat, Thiocyanat, Methylsulfat, Ethylsulfat, Methansulfonat, Formiat, Acetat, Glycolat, Lactat, Dimethylphosphat, Diethylphosphat, p-Tolylsulfonat, Tetrafluoroborat und Hexafluorophosphat, sowie Chlorid, Bromid.

Das gewünschte Anion kann durch Ionenaustausch an bekannten Ionenaustauscher(harzen) eingeführt werden.

Erfindungsgemäß in einer weiteren bevorzugten Ausführungsform werden Imidazolinium-Salze bzw. deren Mischungen verwendet, die Bis(trifluormethylylsulfonyl)imid, Perfluoralkyltosylate, Alkylsulfate und -sulfonate, perfluorierte Alkylsulfonate und -sulfate, Perfluoralkylcarboxylate, Perchlorat, Dicyanamid, Thiocyanat, Isothiocyanat, Tetraphenylborat, Tetrakis(pentafluorphenyl)-borat, Tetrafluoroborat, Hexafluorophosphat enthalten.

Erfindungsgemäß in einer weiteren bevorzugten Ausführungsform werden Imidazolinium-Salze bzw. deren Mischungen verwendet, die halogenidfrei sind.

Ein weiterer Gegenstand dieser Erfindung ist ein Verfahren zur Herstellung der erfindungsgemäßen Produkte. Dieses ist dadurch gekennzeichnet, dass Carbonsäuren bzw. Carbonsäurederivate, wie z.B. Carbonsäureester, mit Aminoethylethanolamin unter Wasserabspaltung zu den entsprechenden Amidaminen umgesetzt werden und anschließend bei gegenüber dem ersten Verfahrensschritt weiter erhöhter Temperatur zum Imidazolin-Derivat zyklisiert werden. Mit oder ohne weitere Aufbereitung werden die Imidazolin-Derivate danach durch Alkylierung in die erfindungsgemäßen Imidazolinium-Salze überführt. Zur Alkylierung können alle dem Fachmann bekannte Alkylierungsmittel, insbesondere Alkylhalogenide, Dialkylsulfate, Alkylsulfonsäureester und Dialkylcarbonate eingesetzt werden. Die erfindungsgemäßen Stoffe nach Formel II werden durch Umsetzung mit Ethylenoxid/Brönsted-Säure quarterniert. Durch Ionenaustausch, beispielweise an Ionenaustauscherharzen, kann jedes beliebige Anion als Gegenion erhalten werden.

Vor der Alkylierung können die Imidazolin-Derivate an der freien Hydroxylgruppe funktionalisiert werden. Alle chemischen Umwandlungen, die den nachfolgenden Alkylierungsschritt nicht beeinträchtigen, sind prinzipiell möglich. Bevorzugt kann die Hydroxylgruppe alkoxyliert, verstert oder amidiert werden.

Mit der erfindungsgemäßen Verwendung von mindestens einem Imidazolinium-Salz wird ein neuartiges Schmiermittel bereitgestellt, das sich durch ein hervorragendes Eigenschaftsprofil, insbesondere durch sehr niedrige Reibungskoeffizienten, auszeichnet.

Gleichzeitig verfügen die Stoffe über ein in weiten Bereichen anpassbares Eigenschaftsprofil im Hinblick auf Viskosität, Dichte, Thermostabilität, anti-korrosive Eigenschaften, Oxidationsstabilität, Materialverträglichkeit, Verschleißschutz, Kälteeignung, VT-Verhalten (Viskositäts-Temperatur-Verhalten), Mischbarkeiten, Hydrolysebeständigkeiten, Toxizität und Ecotoxizität. Auf dieser Basis kann für jede Anwendung ein maßgeschneidertes Schmiermittel bereitgestellt werden, das die Anforderungen bezüglich der vorerwähnten Eigenschaften voll erfüllt. Damit kann mit einem Imidazolinium-Salz beziehungsweise einer Mischung aus Imidazolinium-Salzen als Schmiermittel eine hohe Energieeffizienz ("fuel efficicy") erreicht werden. Darüber hinaus kann ein Schmiermittel eingesetzt werden, das sich beim Feinfiltrierprozess auch bei niedrigen Temperaturen nicht statisch auflädt und somit einen sicheren Betrieb gewährleistet.

Aufgrund der Tatsache, dass einige Imidazolinium-Salze durch ihr Eigenschaftsprofil so ausgewählt werden können, dass sie hochtemperaturbeständig, nicht brennbar, und korrosionshemmend sind und in Bezug auf Viskosität, Dichte, Oxidationsstabilität, Materialverträglichkeit, Verschleißschutz, Kälteeignung, V-T-Verhalten, Mischbarkeiten, und Hydrolysebeständigkeiten exakt an die jeweiligen Spezifikationen angepasst werden können, können diese Imidazolinium-Salze als Schmiermittel vorteilhaft eingesetzt werden. Außerdem zeigen Imidazolinium-Salze keinen Dampfdruck unterhalb ihrer Zersetzungstemperatur und besitzen eine intrinsische elektrische Leitfähigkeit.

Daneben werden besonders bevorzugt solche Imidazolinium-Salze ausgewählt, die biologisch abbaubar und gleichzeitig nicht toxisch sind. Neben den vorgenannten Vorteilen sind gerade diese beiden zusätzlichen Eigenschaften wichtige Kriterien bei der Auswahl von Schmierstoffen für eine Serienanwendung im industriellen Umfeld.

Beispielhafte Vertreter dieser neuartigen Verbindungen sind die in den Beispielen verwendeten Imidazoliniumsalze (1) bis (5)
Imidazoliniumsalz (1): 1-Hydroxyethyl-2,3-dimethylimidazolinium methylsulfat, Schmelzpunkt < 25 °C, Zersetzungstemperatur > 200 °C
Imidazoliniumsalz (2):1-Hydroxyethyl-2,3-diethylimidazolinium ethylsulfat, Schmelzpunkt < 25 °C, Zersetzungstemperatur > 200 °C
Imidazoliniumsalz (3):1-Hydroxethyl-2-capryl-3-methylimidazolinium methansulfonat, Schmelzpunkt < 25 °C, Zersetzungstemperatur > 250 °C
Imidazoliniumsalz (4): 1-Hydroxyethyl-2,3-dimethylimidazolinium dicyanamid, Schmelzpunkt < 25 °C, Zersetzungstemperatur > 250 °C
Imidazoliniumsalz (5) : 1,3-dihydroxyethyl-2-methylimidazolinium acetat, Schmelzpunkt < 25 °C, Zersetzungstemperatur > 250 °C.

Im Vergleich zu den bisherigen Schmierstoffen besitzen die Imidazolinium-Salze über ein sehr breites Temperaturintervall sehr niedrige Reibungskoeffizienten, was bei der Schmierung von Bauteilen zu hoher Energieeffizienz führt. Daneben kann das als Schmierstoff eingesetzte Imidazolinium-Salz ohne statische Aufladung feinfiltriert werden, so dass elektrische Entladungen und damit verbundene unkontrollierbare Gefahren vermieden werden. Zudem kann die Viskosität der Imidazolinium-Salze an das Anforderungsprofil angepasst werden. Darüber hinaus weisen die Imidazolinium-Salze oder die Mischung aus ImidazoliniumSalzen eine deutlich verbesserte Additivierbarkeit, insbesondere mit EP-Additiven zur weiteren Optimierung der speziellen reibungs- und verschleißmindernden Eigenschaften, auf.

Die Auswahl der Imidazolinium-Salze oder der Mischung von Imidazolinium-Salzen richtet sich nach dem Anforderungsprofil der gewählten tribologischen Anwendung.

In einer bevorzugten Ausführungsform der Erfindung ist das geschmierte Aggregat ein Verbrennungsmotor. Gerade bei diesem Prozess kann das Imidazolinium-Salz oder die Mischung von Imidazolinium-Salzen mit ihrem vorstehend erläuterten Eigenschaftsprofil eine große Verbesserung und Vereinfachung erzielen.

Zur Einstellung des jeweiligen Eigenschaftsprofils kann das Imidazolinium-Salz oder die Mischung von Imidazolinium-Salzen mit weiteren Stoffen, insbesondere anderen Salzen, gemischt werden. Bevorzugte Salze sind organische Salze, besonders bevorzugt sind Ammoniumsalze und Imidazoliumsalze.

Zur Einstellung des jeweiligen Eigenschaftsprofils kann das Imidazolinium-Salz oder die Mischung von Imidazolinium-Salzen oder die Mischung von Imidazolinium-Salzen mit weiteren Stoffen, insbesondere anderen Schmierstoffen, gemischt werden. Bevorzugte Schmierstoffe sind Mineralöle, Poly-alpha-Olefine, Esteröle und Naturöle.

Zur Einstellung des jeweiligen Eigenschaftsprofils kann das Imidazolinium-Salz oder die Mischung von Imidazolinium-Salzen oder die Mischung von Imidazolinium-Salzen mit weiteren (Schmier-) Stoffen durch weitere Stoffe additiviert werden. Bevorzugte Additive sind Korrosionsinhibitoren, EP(extreme pressure)-Additive, Viskositätsindexverbesserer, Antioxidationsmittel und Detergentien.

Des Weiteren können zur Einstellung des jeweiligen Eigenschaftsprofils Schmierstoffe durch das Imidazolinium-Salz oder die Mischung von Imidazolinium-Salzen additiviert werden.

Bei Einsatz im wässrigen Medium, kann das Imidazolinium-Salz oder die Mischung von Imidazolinium-Salzen oder die Mischung von Imidazolinium-Salze mit weiteren (Schmier-) Stoffen durch Zugabe eines Puffers stabilisiert werden.

In einer weiteren Ausgestaltung der vorliegenden Erfindung weist das Imidazolinium-Salz oder die Mischung von Imidazolinium-Salzen oder die Mischung von Imidazolinium-Salzen mit weiteren (Schmier-) Stoffen eine Schmelztemperatur unterhalb von 100 °C, bevorzugt von unterhalb 50 °C und besonders bevorzugt von unterhalb 25 °C auf.
Die erfindungsgemäßen Imidazolinium-Salze besitzen selbst schon Schmelzpunkte unterhalb von 150 °C, bevorzugt unterhalb von 100 °C und besonders bevorzugt unterhalb von 25 °C.

Besonders bevorzugt kann das Imidazolinium-Salz oder die Mischung von Imidazolinium-Salzen oder die Mischung von Imidazolinium-Salzen mit weiteren (Schmier-) Stoffen einen Flüssigkeitsbereich von -85 °C bis 400 °C, bevorzugt von -70 °C bis 300 °C und besonders bevorzugt von -60 °C bis 200 °C aufweisen.

Eine weitere bevorzugte Ausführungsform der Erfindung sieht die Verwendung eines Imidazolinium-Salzes oder einer Mischung von Imidazolinium-Salzen oder die Mischung von Imidazolinium-Salzen mit weiteren (Schmier-) Stoffen mit einem Pourpoint von bis zu -65 °C vor.

Die Imidazolinium-Salze oder die Mischung von Imidazolinium-Salzen oder die Mischung von Imidazolinium-Salzen mit weiteren (Schmier-) Stoffen werden verwendet in Mischungen in denen das Imidazoliniumsalz oder deren Mischung 0,1 bis 99,98 Gew.-% der Gesamtmischung ausmacht, bevorzugt 75 bis 99,95 Gew.-%, besonders bevorzugt 85 bis 99,9 Gew.-%.

Zusammenfassend wird demnach eine Verwendung von mindestens einem Imidazolinium-Salz oder von Mischungen aus Imidazolinium-Salzen oder die Mischung von Imidazolinium-Salzen mit weiteren (Schmier-) Stoffen für die Schmierung von Bauteilen vorgeschlagen, aufgrund derer eine erhebliche Vereinfachung und Vorteile bei der Handhabung erreicht werden. So sind zum Beispiel Schmierstoffflecken auf Textilien oder im Motorraum einfach durch Wasser aus- bzw. abwaschbar.

Hinsichtlich der Tatsache, dass die Imidazolinium-Salze der Formel (I), (II) oder (III) durch ihre anpassbaren Eigenschaftsprofile und Mischbarbeit hochtemperaturbeständig, nicht brennbar, korrosionshemmend, oxidationsstabil, verschleißhemmend, scherstabil und additivierbar sind, können sie als Schmiermittel, insbesondere im Verbrennungsmotor vorteilhaft eingesetzt werden. Zudem zeigen Imidazolinium-Salze keinen Dampfdruck unterhalb der Zersetzungstemperatur, können biologisch abgebaut werden und sind nicht toxisch.

Im Vergleich zu den Imidazoliniumsalzen der Formel (I), (II) bzw. (III) zeigen die gängigen Schmiermittel des Standes der Technik wie beispielsweise Poly-alpha-Olefine (PAO"s) oder Mineralöle (z.B. Castrol®, Warenzeichen der Castrol Ltd.) höhere Reibzahlen, eine niedrigere Zersetzungstemperatur, leichte Entflammbarkeit und hohe Brennbarkeit, sowie eine schlechte Umweltverträglichkeit.

Aufgrund ihres Eigenschaftsprofils eignen sich ausgewählte Imidizolinium-Salze für Anwendungen, bei denen die Stoffe kurzfristig oder dauerhaft unter starker thermischer Belastung stehen. Bevorzugte Imidazolinium-Salze oder Mischungen aus Imidazolinium-Salzen zeichnen sich durch eine thermische Stabilität < 200 °C aus. Die Bestimmung der thermischen Stabilität kann auf an sich bekannte Weise erfolgen.

Besonders bewährt hat sich jedoch die Thermogravimetrie, wobei mit einer Shimadzu TG-50 und einer Heizrate von 10°C/min unter Stickstoffatmosphäre (Stickstofffluss: 23 ml/min) gemessen wird.

Unter diesem Bedingungen zeigen thermostabile Imidazolinium-Salze bei 200 °C bevorzugt eine Gewichtsabnahme > 50 %, besonders bevorzugt > 30 %, zweckmäßigerweise > 20 %, noch mehr bevorzugt > 10 % und insbesondere > 5 %, jeweils bezogen auf das Ausgangsgewicht.

Die erfindungsgemäßen Imidazoliniumsalze und deren Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören.

### Ausführungsbeispiele:

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Alle Prozentangaben beziehen sich auf das Gewicht soweit nichts anderes angegeben ist.

Generelle Herstellvorschrift eines Imidazoliniumsalzes ohne Zwischenproduktisolierung (Formel I)

In einem 4-Hals-Glaskolben werden 1,75 Mol Aminoethlethanolamin (AEEA) und 0,01 Mol Na-Methylat 30%ig unter N₂ vorgelegt. 1 Mol des jeweiligen Carbonsäureesters werden bei 70 °C unter schwacher Exothermie binnen 1,5 h zugesetzt, wobei die Temperatur 90 °C nicht übersteigen sollte. Die Amidierung findet binnen 6 bis 10 h statt. Zur Kontrolle wird ein IR-Spektrum bei 1.740 nm aufgenommen. Eine ausreichende Alkalität (> 2 mgKOH/g) muss gewährleistet sein. Nach erfolgter Amidierung wird die Alkalität mittels Phosphorsäure 85 % neutralisiert und anschließend unter Methanol-/Ethanol-Destillation (Kolonne) auf 175 °C zur Cyclisierung aufgeheizt. Vakuum wird angelegt und binnen 3 h auf 60 mbar eingestellt, wobei das Reaktionswasser destilliert. Kontrolle über IR-Spekrum bei 1.600 sowie 1.650 nm. Nach erfolgter Cyclisierung wird der Überschuss AEEA unter Vakuum bei < 20 mbar und 175 °C destilliert. Die analytische Kontrolle erfolgt über GAZ (Gesamtaminzahl) und TAZ (Tertiäre Aminzahl).

Anschließend wird auf 50 °C abgekühlt und 0,99 Mol des Alkylierungsmittels langsam zugetropft. Der Fortgang der Reaktion wird mittels Aminzahl überprüft. Nach 1 bis 24 h ist die Reaktion abgeschlossen. Der verbleibende Rückstand kann je nach Anwendungsgebiet direkt ohne weitere Aufarbeitung erfindungsgemäß eingesetzt werden oder durch dem Fachmann bekannte Reinigungsmethoden aufgereinigt werden.

Generelle Herstellvorschrift eines Imidazoliniumsalzes ohne Zwischenproduktisolierung (Formel II)

In einem 4-Hals-Glaskolben werden 1,75 Mol Aminoethylethanolamin (AEEA) und 0,01 Mol Na-Methylat 30%ig unter N₂ vorgelegt. 1 Mol des jeweiligen Carbonsäureesters werden bei 70 °C unter schwacher Exothermie binnen 1,5 h zugesetzt, wobei die Temperatur 90 °C nicht übersteigen sollte. Die Amidierung findet binnen 6 bis 10 h statt. Zur Kontrolle wird ein IR-Spektrum bei 1.740 nm aufgenommen. Eine ausreichende Alkalität (> 2 mgKOH/g) muss gewährleistet sein. Nach erfolgter Amidierung wird die Alkalität mittels Phosphorsäure 85 % neutralisiert und anschließend unter Methanol-/Ethanol-Destillation (Kolonne) auf 175 °C zur Cyclisierung aufgeheizt. Vakuum wird angelegt und binnen 3 h auf 60mbar eingestellt, wobei das Reaktionswasser destilliert. Kontrolle über IR-Spekrum bei 1.600 sowie 1.650 nm. Nach erfolgter Cyclisierung wird der Überschuss AEEA unter Vakuum bei < 20 mbar und 175 °C destilliert. Die analytische Kontrolle erfolgt über GAZ und TAZ. Anschließend wird unter starker Kühlung 1 Mol einer Brönstedt-Säure so langsam zugetropft, dass die Temperatur von 50 °C nicht überschritten wird. Das Reaktionsende wird über die Aminzahl bestimmt.

Anschließend wird das Reaktionsgefäß evakuiert und mit Stickstoff gefüllt, so dass ein Druck von 0,5 bar eingestellt wird. Nach Zudosierung von 1 Mol Ethylenoxid über 4 Stunden, wird noch 2 Stunden bei 65 °C nachreagiert und danach im Vakuum flüchtige Nebenprodukte abdestilliert. Der verbleibende Rückstand kann je nach Anwendungsgebiet direkt ohne weitere Aufarbeitung erfindungsgemäß eingesetzt werden oder durch dem Fachmann bekannte Reinigungsmethoden aufgereinigt werden.

Generelle Herstellvorschrift eines Imidazoliniumsalzes ohne Zwischenproduktisolierung (Formel III)

In einem 4-Hals-Glaskolben werden 1,75 Mol Aminoethylethanolamin (AEEA) und 0,01 Mol Na-Methylat 30%ig unter N₂ vorgelegt. 1 Mol des jeweiligen Carbonsäureesters werden bei 70 °C unter schwacher Exothermie binnen 1,5 h zugesetzt, wobei die Temperatur 90 °C nicht übersteigen sollte. Die Amidierung findet binnen 6 bis 10 h statt. Zur Kontrolle wird ein IR-Spektrum bei 1.740 nm aufgenommen. Eine ausreichende Alkalität (> 2 mgKOH/g) muss gewährleistet sein. Nach erfolgter Amidierung wird die Alkalität mittels Phosphorsäure 85 % neutralisiert und anschließend unter Methanol-/Ethanol-Destillation (Kolonne) auf 175 °C zur Cyclisierung aufgeheizt. Vakuum wird angelegt und binnen 3 h auf 60mbar eingestellt, wobei das Reaktionswasser destilliert. Kontrolle über IR-Spekrum bei 1.600 sowie 1.650 nm. Nach erfolgter Cyclisierung wird der Überschuss AEEA unter Vakuum bei < 20 mbar und 175 °C destilliert. Die analytische Kontrolle erfolgt über GAZ und TAZ. Anschließend wird auf 50 °C abgekühlt und 0,495 Mol eines difunktionellen Alkylierungsmittels langsam zugetropft. Der Fortgang der Reaktion wird mittels Aminzahl überprüft. Nach 1 bis 24 h ist die Reaktion abgeschlossen. Der verbleibende Rückstand kann je nach Anwendungsgebiet direkt ohne weitere Aufarbeitung erfindungsgemäß eingesetzt werden oder durch dem Fachmann bekannte Reinigungsmethoden aufgereinigt werden.

### Anwendungsbeispiele:

Die anwendungstechnische Austestung erfolgte mit einer Mini-Traction-Machine (MTM2) erhältlich von der Firma PCS-Instruments, Ltd. (London). Damit wurden die Reibungskoeffizenten bei unterschiedlichen Temperaturen, Drücken und Drehgeschwindigkeiten ermittelt und mit marktüblichen Produkten (Mineralöl, Poly-alpha-olefin) verglichen.

Die Imidazoliniumsalze (1) bis (5) entsprechend den vorstehenden Synthesevorschriften wurden auf ihre Eignung als Schmiermittel getestet. Folgende Verbindungen wurden als Imidazoliniumsalz (1) bis (5) ausgetestet:
Imidazoliniumsalz (1): 1-Hydroxyethyl-2,3-dimethylimidazoliniummethylsulfat, Schmelzpunkt < 25 °C, Zersetzungstemperatur > 200 °C
Imidazoliniumsalz (2):1-Hydroxyethyl-2,3-diethylimidazoliniumethylsulfat, Schmelzpunkt < 25 °C, Zersetzungstemperatur > 200 °C
Imidazoliniumsalz (3):1-Hydroxethyl-2-capryl-3-methylimidazoliniummethansulfonat, Schmelzpunkt < 25 °C, Zersetzungstemperatur > 250 °C
Imidazoliniumsalz (4) : 1-Hydroxyethyl-2,3-dimethylimidazoliniumdicyanamid, Schmelzpunkt < 25 °C, Zersetzungstemperatur > 250 °C
Imidazoliniumsalz (5) : 1,3-dihydroxyethyl-2-methylimidazolinium acetat, Schmelzpunkt < 25 °C, Zersetzungstemperatur > 250 °C

**Tabelle 1:**

| Kraft 75N, Geschwindigkeit 1.000 mm/s | | | |
|---|---|---|---|
| | Reibungskoeffizient | | |
| | bei 40 °C | bei 80 °C | bei 110 °C |
| Imidazolinium-Salz (1) | sehr niedrig | niedrig | niedrig |
| Imidazolinium-Salz (2) | sehr niedrig | sehr niedrig | niedrig |
| Imidazolinium-Salz (3) | sehr niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | sehr niedrig | niedrig | niedrig |
| 1:1 Mischung aus Imidazoliniumsalz (1) und 1-Ethyl-3-methylimidazoliumethylsulfat | sehr niedrig | sehr niedrig | sehr niedrig |
| Mineralöl CASTROL | hoch | Hoch | hoch |
| Poly-alpha-olefin PAO 8 | mittel | Mittel | mittel |

**Tabelle 2:**

| Kraft 75N, Geschwindigkeit 100 mm/s | | | |
|---|---|---|---|
| | Reibungskoeffizient | | |
| | bei 40 °C | bei 80 °C | bei 110 °C |
| Imidazolinium-Salz (1) | niedrig | niedrig | niedrig |
| Imidazolinium-Salz (2) | sehr niedrig | sehr niedrig | niedrig |
| Imidazolinium-Salz (3) | niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | niedrig | niedrig | niedrig |
| 1:1 Mischung aus Imidazoliniumsalz (1) und 1-Ethyl-3-methylimidazoliumethylsulfat | sehr niedrig | sehr niedrig | sehr niedrig |
| Mineralöl CASTROL | sehr hoch | sehr hoch | sehr hoch |
| Poly-alpha-olefin PAO 8 | hoch | hoch | hoch |

**Tabelle 3:**

| Kraft 75N, Geschwindigkeit 10 mm/s | | | |
|---|---|---|---|
| | Reibungskoeffizient | | |
| | bei 40 °C | bei 80 °C | bei 110 °C |
| Imidazolinium-Salz (1) | niedrig | mittel | mittel |
| Imidazolinium-Salz (2) | sehr niedrig | sehr niedrig | niedrig |
| Imidazolinium-Salz (3) | niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | niedrig | niedrig | niedrig |
| 1:1 Mischung aus Imidazoliniumsalz (1) und 1-Ethyl-3-methylimidazoliumethylsulfat | sehr niedrig | sehr niedrig | sehr niedrig |
| Mineralöl CASTROL | hoch | sehr hoch | sehr hoch |
| Poly-alpha-olefin PAO 8 | hoch | hoch | sehr hoch |

**Tabelle 4:**

| Kraft 40N, Geschwindigkeit 1.000 mm/s | | | |
|---|---|---|---|
| | Reibungskoeffizient | | |
| | bei 40 °C | bei 80 °C | bei 110 °C |
| Imidazolinium-Salz (1) | sehr niedrig | niedrig | niedrig |
| Imidazolinium-Salz (2) | sehr niedrig | sehr niedrig | niedrig |
| Imidazolinium-Salz (3) | sehr niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | sehr niedrig | niedrig | niedrig |
| 1:1 Mischung aus Imidazoliniumsalz (1) und 1-Ethyl-3-methylimidazoliumethylsulfat | sehr niedrig | sehr niedrig | sehr niedrig |
| Mineralöl CASTROL | mittel | mittel | hoch |
| Poly-alpha-olefin PAO 8 | mittel | mittel | mittel |

**Tabelle 5:**

| Kraft 40N, Geschwindigkeit 100 mm/s | | | |
|---|---|---|---|
| | Reibungskoeffizient | | |
| | bei 40 °C | bei 80 °C | bei 110 °C |
| Imidazolinium-Salz (1) | niedrig | niedrig | sehr niedrig |
| Imidazolinium-Salz (2) | sehr niedrig | sehr niedrig | niedrig |
| Imidazolinium-Salz (3) | sehr niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | sehr niedrig | niedrig | niedrig |
| 1:1 Mischung aus Imidazoliniumsalz (1) und 1-Ethyl-3-methylimidazolium ethylsulfat | sehr niedrig | sehr niedrig | sehr niedrig |
| Mineralöl CASTROL | mittel | mittel | hoch |
| Poly-alpha-olefin PAO 8 | mittel | mittel | mittel |

**Tabelle 6:**

| Kraft 40N, Geschwindigkeit 10 mm/s | | | |
|---|---|---|---|
| | Reibungskoeffizient | | |
| | bei 40 °C | bei 80 °C | bei 110 °C |
| Imidazolinium-Salz (1) | niedrig | niedrig | niedrig |
| Imidazolinium-Salz (2) | sehr niedrig | sehr niedrig | sehr niedrig |
| Imidazolinium-Salz (3) | niedrig | niedrig | niedrig |
| Imidazolinium-Salz (4) | sehr niedrig | niedrig | niedrig |
| Imidazolinium-Salz (5) | niedrig | niedrig | niedrig |
| 1:1 Mischung aus Imidazoliniumsalz (1) und (3) | niedrig | niedrig | niedrig |
| 1:1 Mischung aus Imidazoliniumsalz (1) und 1-Ethyl-3-methylimidazoliumethylsulfat | sehr niedrig | sehr niedrig | sehr niedrig |
| Mineralöl CASTROL | hoch | hoch | hoch |
| Poly-alpha-olefin PAO 8 | mittel | mittel | hoch |

Die Ergebnisse zeigen, dass die erfindungsgemäßen Imidazolinium-Salze deutlich niedrige Reibungskoeffizienten unter gleichen Versuchsbedingungen aufweisen als die Schmiermittel nach dem Stand der Technik. Das erlaubt eine Reduzierung der Reibungsverluste und eine Verbesserung der Energiebilanz ("Fuel efficiency").

## Patentansprüche

1. Imidazoliniumsalze der allgemeinen Formeln (I), (II) oder (III), wobei Formel (I) 1-Hydroxyalkyl- und 1-Alkoxyalkyl-imidazolinium-Salzen entspricht, 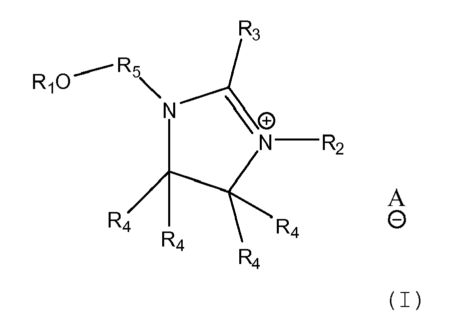 wobei
R₁ Wasserstoff oder ein gegebenenfalls verzweigtes, gegebenenfalls ungesättigtes, gegebenenfalls Sauerstoffatome oder Stickstoffatome enthaltendes Alkylradikal mit 1 bis 20 C-Atomen,
R₂ Wasserstoff oder ein gegebenenfalls verzweigtes, gegebenenfalls ungesättigtes, gegebenenfalls Sauerstoffatome oder Stickstoffatome enthaltendes Alkylradikal mit 1 bis 20 C-Atomen,
R₃ ein gegebenenfalls verzweigtes, gegebenenfalls ungesättigtes, gegebenenfalls von Sauerstoffatomen unterbrochenes Alkylradikal mit 1 bis 20 C-Atomen,
R₄ jeweils unabhängig voneinander Wasserstoff oder Alkyl, bevorzugt Butyl, Propyl, Ethyl, Methyl oder Wasserstoff,
R₅ ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Ethylen, ist und
A ein Gegenion zu den positiven Ladungen an den quartären Stickstoffgruppen sind,
sowie 1,3-Dihydroxyalkyl-imidazolinium- und 1-Alkoxyalkyl-3-hydroxy-imidazolinium-Salze der allgemeinen Formel (II) entsprechen, 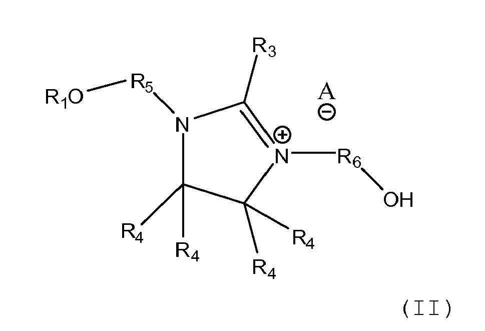 wobei
R₁, R₃, R₄ und R₅ die unter Formel (I) genannten Bedeutungen besitzen und
R₆ ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Ethylen, ist und
A ein Gegenion zu den positiven Ladungen an den quartären Stickstoffgruppen ist,
sowie der allgemeinen Formel (III) 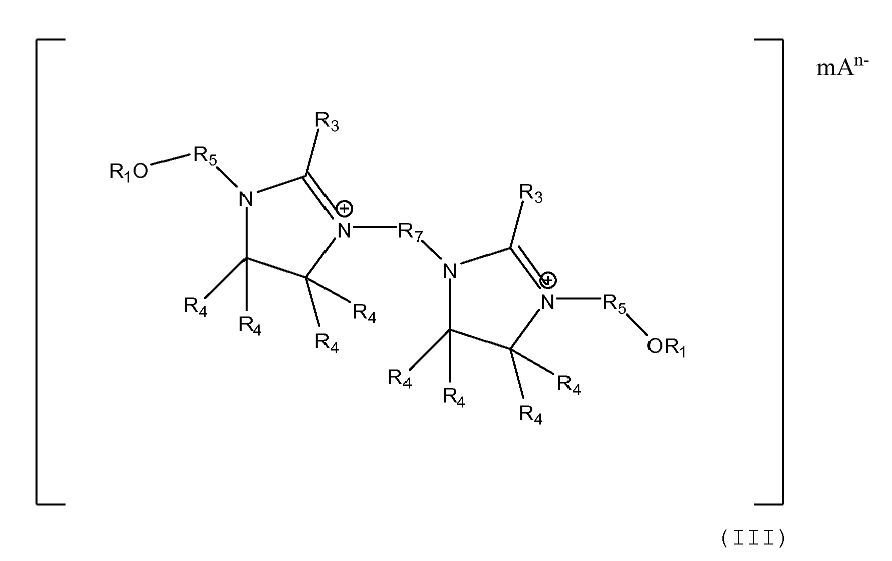 wobei
R₁, R₃, R₄ und R₅ die unter Formel (I) genannten Bedeutungen besitzen und
R₇ ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest, bevorzugt Butylen, Propylen, Ethylen, Methylen, besonders bevorzugt, Butylen und Ethylen, ist,
A ein Gegenanion zu den positiven Ladungen an den quartären Stickstoffgruppen ist und die Multiplikation aus m und n gleich der Ladung des Imidazoliniumkations entspricht.

2. Imidazolinium-Salze nach Anspruch 1 **dadurch gekennzeichnet, dass** R₃ ein gegebenenfalls verzweigtes Alkylradikal mit 1 bis 20 C-Atomen, R₁ Wasserstoff, R₄ Wasserstoff und R₂ ein gegebenenfalls verzweigtes Alkylradikal mit 1 bis 20 C-Atomen bedeuten.

3. Imidazolinium-Salze nach Anspruch 1 **dadurch gekennzeichnet, dass** R₃ ein gegebenenfalls verzweigtes Alkylradikal mit 1 bis 12 C-Atomen, R₁ Wasserstoff, R₄ Wasserstoff und R₂ ein Alkylradikal mit 1 bis 12 C-Atomen bedeuten.

4. Imidazolinium-Salze nach Anspruch 1 bis 3 **dadurch gekennzeichnet, dass** das Gegenion [A]ⁿ⁻ kein Halogenid ist.

5. Imidazolinium-Salze nach Anspruch 1 bis 4 **dadurch gekennzeichnet, dass** der Schmelzpunkt unterhalb von 150 °C liegt.

6. Verfahren zur Herstellung von Imidazoliniumsalzen nach einem der Ansprüche 1 bis 5, **dadurch gekennzeichnet, dass** Carbonsäuren bzw. Carbonsäurederivate mit Aminoethylethanolamin unter Wasserabspaltung zu den entsprechenden Amidaminen umgesetzt werden, anschließend bei erhöhter Temperatur gegenüber dem ersten Verfahrensschritt zum Imidazolin-Derivat zyklisiert werden und danach durch Alkylierung in die erfindungsgemäßen Imidazolinium-Salze überführt werden.

7. Verfahren zur Herstellung von Imidazoliniumsalzen nach Anspruch 6, **dadurch gekennzeichnet, dass** keine Zwischenprodukte isoliert werden.

8. Verfahren zur Herstellung von Imidazoliniumsalzen nach Anspruch 6, **dadurch gekennzeichnet, dass** das Imidazolin-Derivat an der freien Hydroxylgruppe chemisch funktionalisiert wird, bevor die Alkylierung stattfindet.

9. Verfahren zur Herstellung von Imidazoliniumsalzen nach Anspruch 8, **dadurch gekennzeichnet, dass** die chemische Funktionalisierung eine Alkoxylierung oder eine Veresterung oder eine Amidierung ist.

10. Verfahren zur Herstellung der Imidazoliniumsalze nach Anspruch 8, **dadurch gekennzeichnet, dass** zur Alkylierung Alkylierungsmittel, insbesondere Alkylhalogenide, Dialkylsulfate, Alkylsulfonsäureester und Dialkylcarbonate eingesetzt werden.

11. Verfahren zur Herstellung der Imidazoliniumsalze nach Anspruch 8, **dadurch gekennzeichnet, dass** die erfindungsgemäßen Stoffe durch Umsetzung mit Ethylenoxid/Brönsted-Säure quarterniert werden.

12. Verfahren zur Herstellung der Imidazoliniumsalze nach Anspruch 8, **dadurch gekennzeichnet, dass** das Anion [A]ⁿ⁻ durch Ionenaustausch erhalten wird.

13. Verwendung mindestens eines Imidazolinium-Salzes nach einem der Ansprüche 1 bis 6 oder einer Mischung von Imidazolinium-Salzen oder einer Mischung von Imidazolinium-Salzen mit weiteren (Schmier-) Stoffen als Schmiermittel.

14. Verwendung mindestens eines Imidazolinium-Salzes nach einem der Ansprüche 1 bis 6 oder einer Mischung von Imidazolinium-Salzen oder einer Mischung von Imidazolinium-Salzen mit weiteren (Schmier-) Stoffen als Schmiermittel nach Anspruch 13 **dadurch gekennzeichnet, dass** das Schmiermittel in Verbrennungsmotoren eingesetzt wird.

15. Verwendung mindestens eines Imidazolinium-Salzes nach einem der Ansprüche 1 bis 6 oder einer Mischung von Imidazolinium-Salzen oder einer Mischung von Imidazolinium-Salzen mit weiteren (Schmier-) Stoffen als Schmiermittel nach Anspruch 13 **dadurch gekennzeichnet, dass** das Schmiermittel in Getrieben eingesetzt wird.

16. Verwendung mindestens eines Imidazolinium-Salzes nach einem der Ansprüche 1 bis 6 oder einer Mischung von Imidazolinium-Salzen oder einer Mischung von Imidazolinium-Salzen mit weiteren (Schmier-) Stoffen als Schmiermittel nach Anspruch 13 **dadurch gekennzeichnet, dass** das Schmiermittel als Hydrauliköl eingesetzt wird.

17. Verwendung nach einem der Ansprüche 14 bis 16, **dadurch gekennzeichnet, dass** die Imidazolinium-Salze oder die Mischung von Imidazolinium-Salzen oder die Mischung von Imidazolinium-Salzen mit weiteren (Schmier-) Stoffen EP-Additive zur Optimierung der reibungs- und verschleißmindernden Eigenschaften enthält.

18. Verwendung nach einem der Ansprüche 14 bis 16, **dadurch gekennzeichnet, dass** die Imidazolinium-Salze oder die Mischung von Imidazolinium-Salzen oder die Mischung von Imidazolinium-Salzen mit weiteren (Schmier-) Stoffen Korrosionsschutzmittel, Antioxidationsmittel, Viskositätsindexverbesserer, Detergentien, andere Schmierstoffe, andere Salze enthält, bevorzugt organische Salze, besonders bevorzugt Ammoniumsalze oder Imidazoliumsalze enthält.

19. Verwendung nach einem der Ansprüche 14 bis 18, **dadurch gekennzeichnet, dass** die Imidazolinium-Salze oder die Mischung von Imidazolinium-Salzen oder die Mischung von Imidazolinium-Salzen mit anderen Additiven in wässrigen Medien eingesetzt werden und durch Puffer stabilisiert sind.

20. Verwendung nach den Ansprüchen 14 bis 18, **dadurch gekennzeichnet, dass** das Imidazolinium-Salz oder die Mischung von Imidazolinium-Salzen oder die Mischung von Imidazolinium-Salzen mit weiteren (Schmier-) Stoffen 0,1 bis 99,98 Gew.-% der Gesamtmischung ausmacht.

21. Verwendung nach einem der Ansprüche 14 bis 18, **dadurch gekennzeichnet, dass** das Imidazolinium-Salz oder die Mischung von Imidazolinium-Salzen oder die Mischung von Imidazolinium-Salzen mit weiteren (Schmier-) Stoffen einen Flüssigkeitsbereich von -85 °C bis 400 °C aufweist.
